# EUROPEAN PATENT APPLICATION

(11) **EP 3 178 426 A1**
(43) Date of publication of application: **14.06.2017**
(21) Application number: 16203133.0
(22) Date of filing: 09.12.2016
(51) Int. Cl.: A61B 17/88, A61B 17/34, A61F 2/30

(54) **DELIVERY DEVICE FOR VERTEBROPLASTY AND KYPHOPLASTY PROCEDURES**

(30) Priority: 09.12.2015 US 201562265079 P
(71) Applicant: Dean, Laurian Mark, Columbus, OH 43215 (US)
(72) Inventor: Dean, Laurian Mark, Columbus, OH 43215 (US)
(74) Representative: Conroy, John

(57) **Abstract**

A delivery device (18) includes an elongate cylindrical portion (28) and a spreader portion (30) extending from the cylindrical portion, which can be configured to spread a chemotherapy powder or other medicament within a vertebral bone or other site. The delivery device can optionally be part of a kyphoplasty system and used in a kyphoplasty method for treating cancer.

## Description

### TECHNICAL FIELD

This document relates to surgical procedures and related devices, and more particularly to procedures such as vertebroplasty and kyphoplasty.

### BACKGROUND

Vertebroplasty and kyphoplasty are medical spinal procedures in which bone cement is injected (percutaneously) through a small cannula into a fractured vertebra with the goal of relieving back pain caused by vertebral compression fractures. In vertebroplasty, bone cement is often injected into the collapsed or fractured vertebra. The needle is placed with fluoroscopic x-ray guidance. The cement (commonly PMMA) quickly hardens and forms a support structure within the vertebra that provide stabilization and strength. The needle makes a small puncture in the patient's skin that is covered with a bandage after the procedure.

Kyphoplasty is a variation of a vertebroplasty, often used to restore the height and angle of kyphosis of a fractured vertebra (of certain types), followed by its stabilization using injected bone cement. The procedure typically includes the use of a small balloon that is inflated in the vertebral body to create a void within the cancellous bone prior to cement delivery. Once the void is created, the procedure typically continues in a similar manner as a vertebroplasty, with the bone cement typically delivered directly into the newly created void.

### SUMMARY

Some embodiments of system can include a delivery device configured to direct a chemotherapy medicine to a targeted region of a bone, optionally, as part of kyphoplasty procedure at a vertebral bone. Particular embodiments of the delivery device can be configured for delivering and spreading a chemotherapy drug (such as a predetermined quantity of a chemotherapy powder) to a wall of a bore formed in the bone. In some embodiments, the delivery device can include a spreader portion that operates as a spatula component configured for spreading chemotherapy drug to cancellous bone in the vertebral body. For example, the delivery device can include a spreader portion extending distally from a cylinder portion, optionally at an outwardly angled orientation relative to a central axis of the cylinder portion. Various embodiments can be configured with one or more particular shapes and dimensions.

In one aspect, a chemotherapy delivery device can include an elongate cylindrical portion and a spatula portion extending from the cylindrical portion.

Some of the implementations described herein may optionally include one or more of the following features. The delivery device can be configured to spread chemotherapy drug on tissue in a target site in a bone, for example, along a bore wall of a pilot hole formed into a vertebral body. The spreader portion (sometimes referred to as a "spatula portion" in particular embodiments) can be angled outward with respect to a centerline axis of the cylindrical portion by an angle of about 3-degrees to about 15-degrees, and preferably about 5-degrees in a number of embodiments described herein. The spatula portion can be angled outwardly away from the centerline axis of the cylindrical portion so as to direct a chemotherapy powder or other medicament to a sidewall of a pilot hole or other bore when the delivery device is rotated about the centerline axis. Optionally, one or more edges of the spatula portion are tapered, for example, along a distal tip edge. A system can include the delivery device (as described below), a balloon device configured to operate in a kyphoplasty procedure, and a chemotherapy drug suitable for being spread by the delivery device. Additionally, the system may optionally include bone cement and an instrument to deliver the bone cement, an access needle, a working corridor instrument, a plunger instrument configured to slide within the chemotherapy delivery device, and a drill instrument configured to form a pilot hole in a vertebral bone. The system can be a kyphoplasty system configured for performing a kyphoplasty procedure.

In another aspect, a system can include a means for treating cancer cells, a means for delivering the means for treating cancer cells to a bone such as a vertebral body, and a means for spreading the means for treating cancer cells within a targeted site of the vertebral body.

Some of the implementations described herein may optionally include one or more of the following features. The system may further include a means for simultaneously expanding a hole in a vertebra and pressing the means for treating cancer cells into cancellous bone. Also, the system may include a means for filling the hole in the vertebra.

In one aspect, a method includes delivering chemotherapy drug to a target site in a bone and spreading the chemotherapy drug on bone tissue.

In another aspect, a method comprising delivering chemotherapy drug through an above-described spatula to a target site in a bone and spreading the chemotherapy drug on bone tissue via the spatula.

Some or all of the embodiments described herein may provide one or more of the following advantages. First, some embodiments of the system can deliver chemotherapy drug to a targeted site within bone tissue. This can allow for improved treatment of cancer at the targeted site, for example, along a pilot hole formed within a vertebral bone proximate to a tumor, with less exposure of the chemotherapy drug to other non-targeted tissue. Second, some embodiments can include a delivery device that directs a chemotherapy powder onto a targeted bore wall formed in cancellous bone (and optionally spreads the chemotherapy powder in a generally even manner along the bore wall) after it has been injected. A spatula can be configured with one or more shapes or features to suitably spread the chemotherapy drug such that it can better contact and treat tissue. Third, some embodiments of the invention can utilize an inflatable balloon device after insertion of the chemotherapy drug to further pressure the chemotherapy drug into the cancellous bone at the targeted site (e.g., after the delivery device spread the chemotherapy drug along the wall of the bore that receives the balloon device). Such a feature can further improve contact and saturation between the chemotherapy drug and the target tissue to enhance the chemotherapy treatment.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIGS. 1A-1E are schematic partial sectional views of a vertebra and a system configured to direct a medicament to a targeted region of the vertebra, as viewed in a direction from cephalad to caudal.
FIG. 2A is a schematic perspective view of a delivery device for spreading a chemotherapy drug along a bore wall of a pilot hole formed into a vertebral bone.
FIG. 2B is a schematic lateral view of two vertebrae, with a delivery device for delivering and spreading a chemotherapy drug or other medicament within one of the vertebrae.
FIG. 3A is a perspective view of one embodiment of a delivery device including a spreader portion configured to direct a chemotherapy powder or other medicament.
FIG. 3B is a side view of the delivery device of FIG. 3A.
FIG. 4 is a perspective view of another embodiment of a delivery device including a spreader portion configured to direct a chemotherapy powder or other medicament.
FIGS. 5-7 are side views of a distal end portion of the delivery device of FIG. 4.
FIG. 8 is an axial view of the distal end portion of the delivery device of FIG. 4.
FIG. 9 is a perspective view of a proximal end portion of the delivery device of FIG. 4.
FIG. 10 is a perspective view of one embodiment of a plunger instrument configured for use with a delivery device.

Like reference symbols in the various drawings indicate like elements.

### DETAILED DESCRIPTION

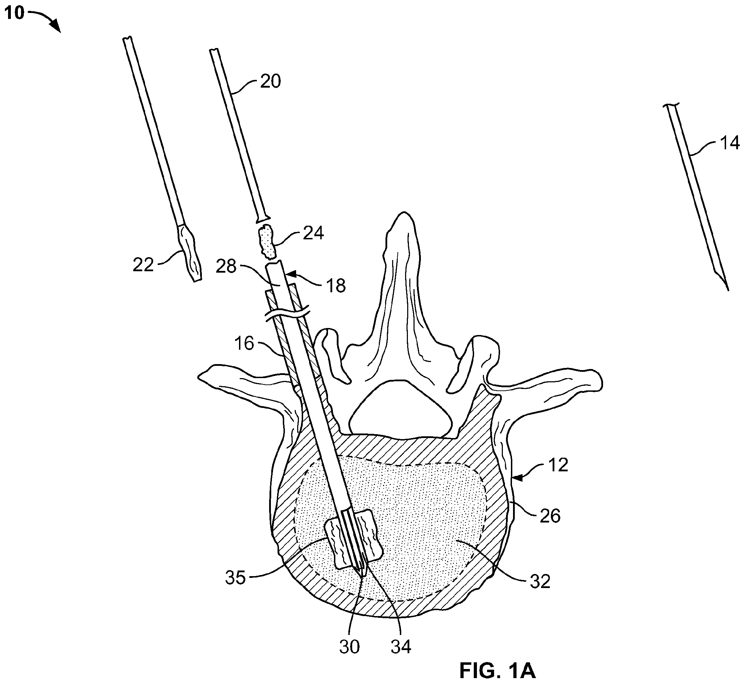

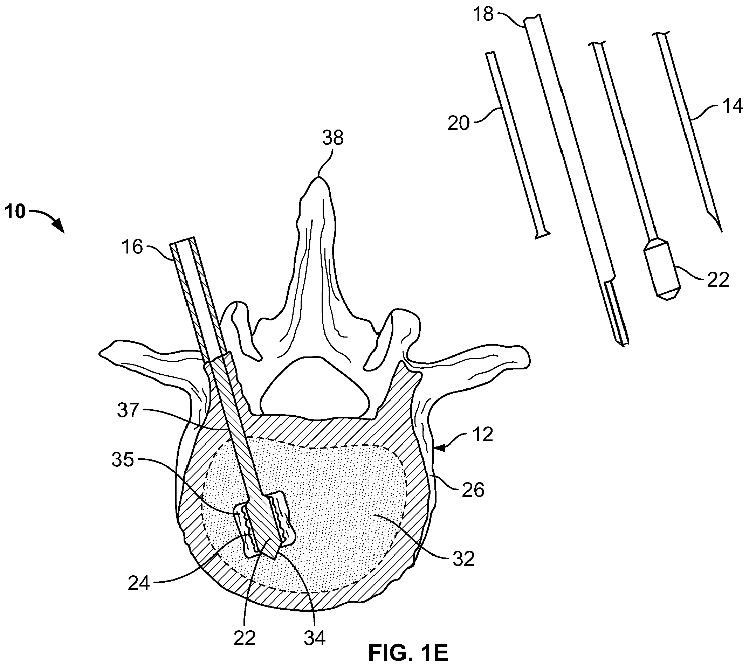

Referring to FIGS. 1A-1E, some embodiments of a system 10 of medical devices are shown along with a partial sectional view of a vertebra 12. In some embodiments, system 10 can include some or all of the following: a needle 14, a cannula 16, a delivery device 18 for delivering and spreading a medicament within the vertebra 12, a plunger 20, a balloon device 22, and a chemotherapy drug 24. The system 10 can be configured for performing vertebroplasty, such as kyphoplasty. The chemotherapy drug 24 can be inserted through the delivery device 18 via the plunger 20 to an interior of a vertebral body 26 of the vertebra 12. The delivery device 18 can be rotated about its longitudinal axis so that a spreader portion at its distal tip directs and spreads the chemotherapy drug 24 (e.g., a chemotherapy powder in this embodiment) against bone in the vertebral body 26, for example, along a wall of a pilot hole formed in the vertebral body 26. The balloon device 22 can then be inserted into the bore of the pilot hole (e.g., after the delivery device 18 has directed the chemotherapy drug to, and spread it along, the wall of the bore of the pilot hole) and inflated to further press the chemotherapy drug 24 into the bone of the vertebral body 26. Inflation of the balloon device 22balloon device 22 can also create a larger cavity in the vertebral body 26 (e.g., for purposes of restoring a height or angle of the vertebral body 26), which can then be filled with bone cement to strengthen and stabilize the vertebral body 26. As such, the system 10 can deliver and apply the chemotherapy drug 24 to localized bone tissue so as to treat cancer in that tissue.

In some embodiments, the needle 14 can be an access needle for helping form an access corridor to the vertebral body 26. The cannula 16 can be an outer cannula and can function as a working corridor instrument. In this embodiment, the delivery device 18 can be configured to operate as an intraosseous cylinder spatula/spreader having a cylinder portion 28 and a spreader portion 30 (also referred to as a spatula portion or blade) extending from a distal end of the cylinder portion 28. The spreader portion 30 can extend from the cylinder portion 28 at an angle to better facilitate spreading of the chemotherapy drug 24 into the cancellous bone 32. The plunger 20 can be sized and configured to press the chemotherapy drug 24 through the cylinder portion 28 of the delivery device 18 to the spreader portion 30 of the delivery device 18. The balloon device 22 can be a kyphoplasty balloon for creating an enlarged cavity in cancellous bone 32 of the vertebral body 26. The chemotherapy drug 24 can be a regional drug for treating cancer in bone, such as a chemotherapy powder.

The chemotherapy drug 24 can be selected to be suitable for the application for a particular patient and that patient's condition. For example, in some embodiments the chemotherapy drug 24 can include bortezomib, also known by the brand name VELCADE. For example, bortezomib can be used in patients with myeloma disease or other suitable conditions. In some embodiments, the chemotherapy drug 24 can include carfilzomib, also known by the brand name KYPROLIS. For example, carfilzomib can be used in patients with relapsed/refractory myeloma or other suitable conditions. In some embodiments, the chemotherapy drug 24 can include doxorubicin HCl liposome injection, also known by the brand name DOXIL. For example, bortezomib can be used in patients with relapsed/refractory myeloma or other suitable conditions, possibly in combination with bortezomib. In some embodiments, the chemotherapy drug 24 can include other types of chemotherapy drugs that are suitable to treat myeloma, such as melphalan and cyclophosphamide.

In some embodiments, the chemotherapy drug 24 can include one or more of the following: bevacizumab (Avastin), Afatinib (Gilotrif), Erlotinib (Tarceva), Gefitinib (Iressa), crizotinib (Xalkori), Docetaxel (Docefrez, Taxotere), Erlotinib, Gefitinib, Pemetrexed, Abraxane (chemical name: albumin-bound or nab-paclitaxel), Adriamycin (chemical name: doxorubicin), carboplatin (brand name: Paraplatin), Cytoxan (chemical name: cyclophosphamide), daunorubicin (brand names: Cerubidine, DaunoXome), Doxil (chemical name: doxorubicin), Ellence (chemical name: epirubicin), fluorouracil (also called 5-fluorouracil or 5-FU; brand name: Adrucil), Gemzar (chemical name: gemcitabine), Halaven (chemical name: eribulin), Ixempra (chemical name: ixabepilone), methotrexate (brand names: Amethopterin, Mexate, Folex), Mitomycin (chemical name: mutamycin), mitoxantrone (brand name: Novantrone), Navelbine (chemical name: vinorelbine), Taxol (chemical name: paclitaxel), Taxotere (chemical name: docetaxel), thiotepa (brand name: Thioplex), vincristine (brand names: Oncovin, Vincasar PES, Vincrex), Xeloda (chemical name: capecitabine), AT: Adriamycin and Taxotere, AC ± T: Adriamycin and Cytoxan, with or without Taxol or Taxotere, CMF: Cytoxan, methotrexate, and fluorouracil, CEF: Cytoxan, Ellence, and fluorouracil, FAC: fluorouracil, Adriamycin, and Cytoxan, CAF: Cytoxan, Adriamycin, and fluorouracil, TAC: Taxotere, Adriamycin, and Cytoxan, GET: Gemzar, Ellence, and Taxol, Herceptin (chemical name: trastuzumab), Revlimid (lenalidomide), Pomalyst (pomalidomide), Thalomid (thalidomide), Farydak (Panobinostat), Revlimid-Velcade-dex (RVD), Velcade-cyclophosphamide-dex (VCD or CyBorD), Velcade-Thalomid-dex (VTD), Revlimid-dex (Rd), and Velcade-dex (Vd).

In other embodiments, the delivery device 18 can be used to apply one or more other drugs suitable for the application, in addition to or instead of a chemotherapy drug.

FIGS. 1A-1E illustrate some of the steps of an example procedure that can be performed with the system 10. Initially, a working channel can be formed into an interior of the vertebral body 26 via the needle 14. The needle 14 can be inserted through the cannula 16, with both introduced simultaneously to or into the vertebra 12. The needle 14 can be removed, leaving the cannula 16 in place to form a working corridor for other instruments to access the vertebral body 26. A bone drill (not shown) can then be inserted through the cannula 16 to drill a hole 34 (e.g., a pilot hole or the like) deeper into bone of the vertebral body 26 as well as a tumor 35 (such as a metastatic bone tumor) that has formed in the vertebral body 26. The drill can then be removed, leaving a hole in the vertebral body, which can extend into the cancellous bone 32.

In some embodiments, the drill can cut a core out of the bone during this procedure. At least some of the core of bone can be replaced to plug the hole after treatment.

The delivery device 18 can be inserted through the cannula 16 and into the bore of the pilot hole 34 formed by the drill. FIG. 1A shows the delivery device 18 extending into the vertebral body 26. As illustrated in FIG. 1A, the delivery device 18 is positioned with the spatula portion 30 located in the hole 34 and the cylinder portion 28 extending out of the vertebral body 26 in a posterior or posterolateral direction.

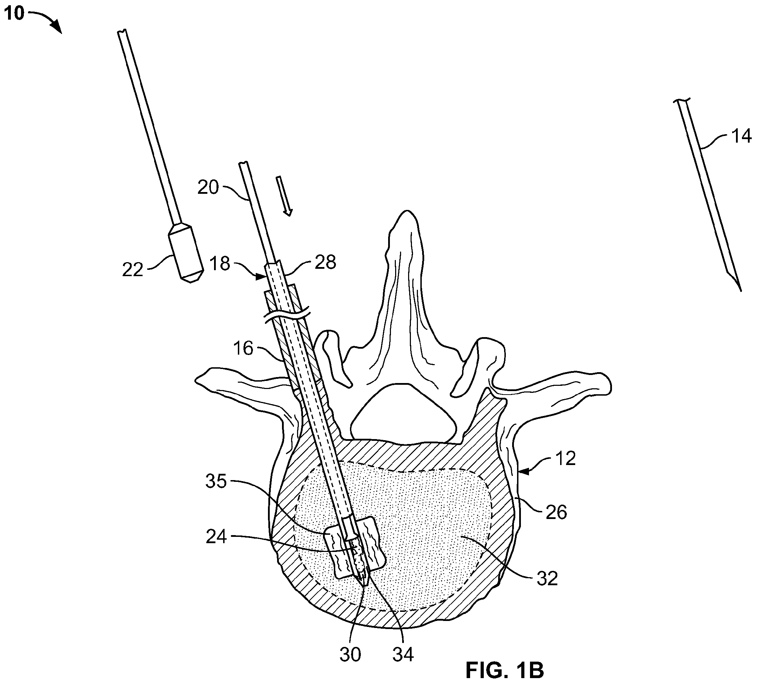

The chemotherapy drug 24 can then be added through the delivery device 18. FIG. 1A is shown with the chemotherapy drug 24 and the plunger 24 aligned with the delivery device 18 and positioned at a proximal end of the cylinder portion 28 of the delivery device 18. The chemotherapy drug 24 can be added to the cylinder portion 30 of the delivery device 18 via a device suitable for the application, such as a syringe. The chemotherapy drug 24 can be inserted into an opening at the proximal end of the cylinder portion 28 and pushed into the hole 34 via the plunger 20. The spatula portion 30 can be curved to form a cavity (also referred to as a scoop) defined by its radially inner surface and a spreading device using its radially outer surface. FIG. 1B shows the plunger 20 extending through the cylinder portion 28 to position the chemotherapy drug 24 next to the spatula portion 30. The plunger 20 can then be removed.

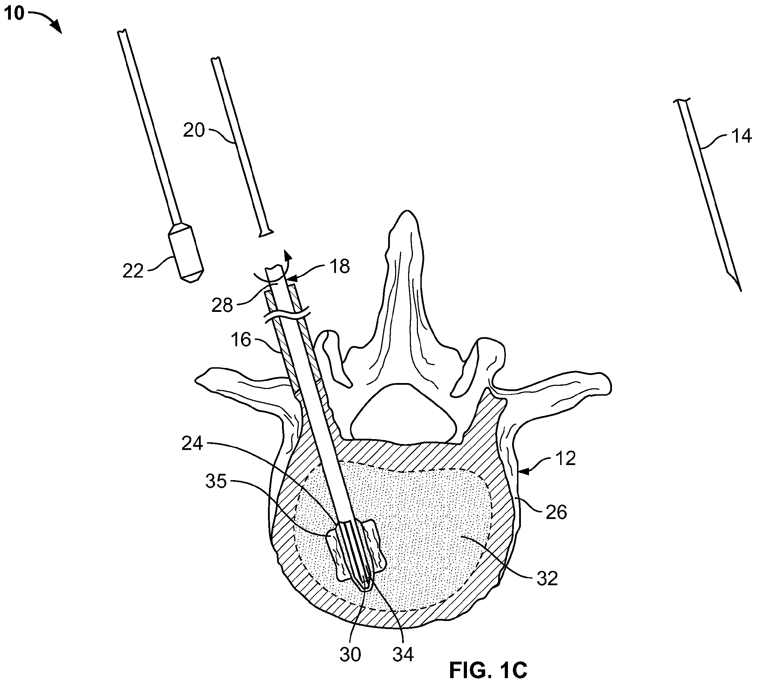

Once the chemotherapy drug 24 is positioned in the hole 34 next to the spatula portion 30, the delivery device 18 can then be rotated to spread the chemotherapy drug 24 against the cancellous bone 32. In some embodiments, the delivery device 18 can be rotated with the plunger 20 still positioned in the delivery device 18. In other embodiments, the plunger 20 can be removed before rotating the delivery device 18. The cylinder portion 28 can be gripped by a hand of a surgeon or other operator and turned, which can consequently turn the spatula portion 30 in the hole 34. The spatula portion 30 can be shaped and configured to facilitate spreading of the chemotherapy drug 24 against the cancellous bone 32 to create better contact and better treat cancer cells in the vertebra 12. FIG. 1C shows the delivery device 18 being rotated, with the chemotherapy drug 24 spread against the cancellous bone 32 in the hole 34.

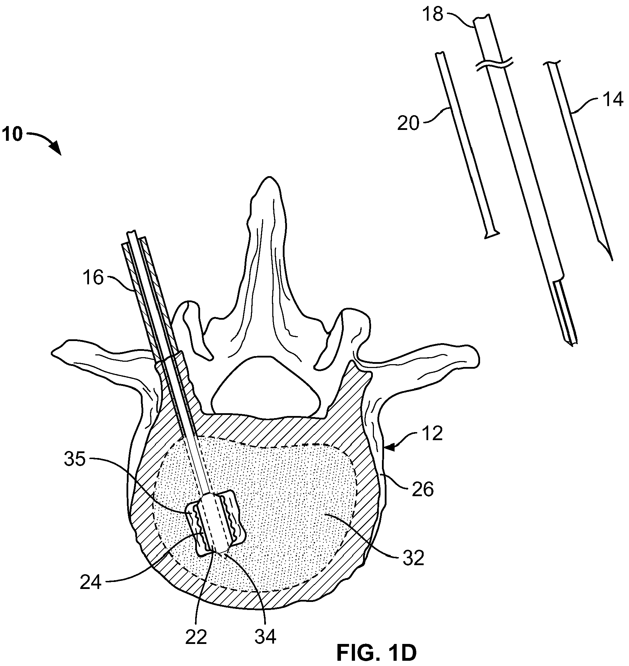

After the chemotherapy drug 24 has been spread, the delivery device 18 can be removed. The balloon 20 can then be inserted through the cannula 16 into the hole 34. Once positioned in the vertebral body 26, the balloon 20 can be inflated. Inflation of the balloon 20 can perform multiple functions. Inflation of the balloon 20 can expand and enlarge the hole 34. In applications where the vertebral body 26 is fractured, inflation of the balloon 20 can restore height and return the vertebral body 26 to a correct or improved position. Inflation of the balloon 20 can also press the chemotherapy drug 24 into the cancellous bone 32. Pressing the chemotherapy drug 24 into the cancellous bone 32 can improve contact with the cancellous bone 32 and better facilitate chemotherapy treatment at localized tissue. FIG. 1D shows the balloon 20 partially expanded and pressing the chemotherapy drug 24 into the cancellous bone 32 and the balloon 20 inflates and enlarges the cavity 34.

After the balloon 20 has been suitable inflated, it can be deflated and removed from the cavity 34 and the cannula 16. The cavity 34 can be filled partially or entirely with bone cement 36, which can be hardened and help stabilize the vertebral body 26. The bone cement can be injected via the plunger 20, via an injection needle (not shown), or via another injector suitable for the application. The bone cement 36 can, in some embodiments, fill both the cavity 34 as well as a pilot hole 37 that was drilled through the vertebra 12 for access to the tumor 35. FIG. IE is shown after the bone cement 36 has been injected into the vertebra 12, and with the bone cement 36 filling some or all of both the cavity 34 and the pilot hole 37. The cannula 16 can then be removed from the patient, the incision can be closed, and the patient can be allowed to heal.

In embodiments in which the drill drilled a core of bone, that core can then be replaced to plug the hole after application of the bone cement and prior to closing the incision.

In some applications, the procedure can be repeated twice for the vertebra 12: once coming from an angle to the right of a spinous process 38 of the vertebra 12 and once coming from an angle to the left of the spinous process 38. One embodiment of the procedure has been shown along the right of the spinous process 38 in FIGS. 1A-1E. A second procedure to the left of the spinous process 38 can be the same or similar to that described herein. By injecting and spreading the chemotherapy drug 24 into both the left and right sides of the vertebral body 26, the chemotherapy drug 24 can better target affected tissue.

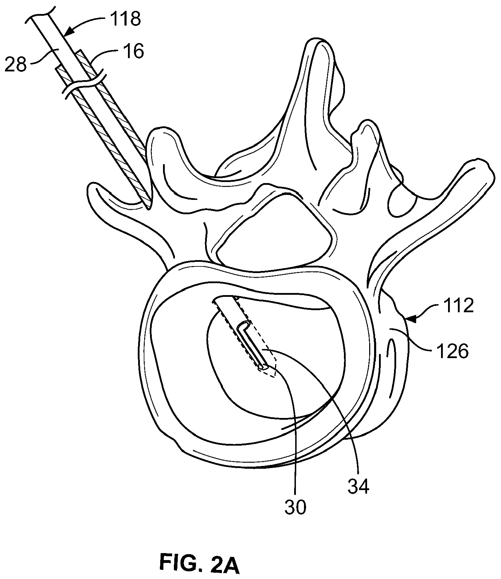

FIG. 2A is a schematic perspective view of a vertebra 112 and its vertebral body 126 with a delivery device 118 extending therein. The delivery device 118 can be the same or similar to the delivery device 18 that is illustrated and described with respect to FIGS. 1A-1E. The center of the vertebral body 126 is illustrated as hollow for clarity.

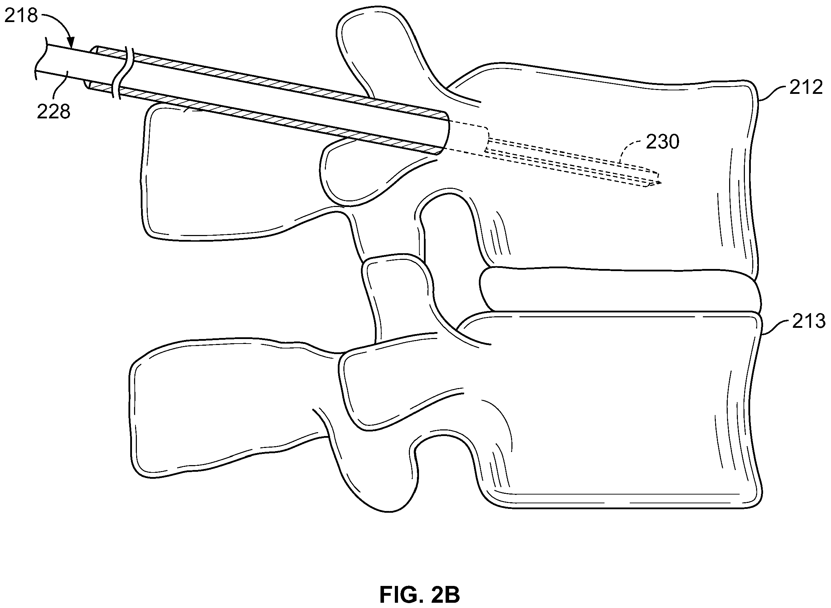

FIG. 2B is a schematic lateral view of two vertebrae 212 and 213, with a delivery device 218 extending into the upper vertebra 212. The delivery device 218 can be the same or similar to the delivery devices 18 (shown in FIGS. 1A-1E) and 118 (shown in FIG. 2A), and can be used in conjunction with other devices in the system 10 (shown in FIGS. 1A-1E). For example, the delivery device 218 can include a cylinder portion 228 and a spreader portion 230. FIGS. 2A and 2B illustrate possible positions and orientations of the delivery devices 118 and 218 with respect to vertebrae 112 and 212.

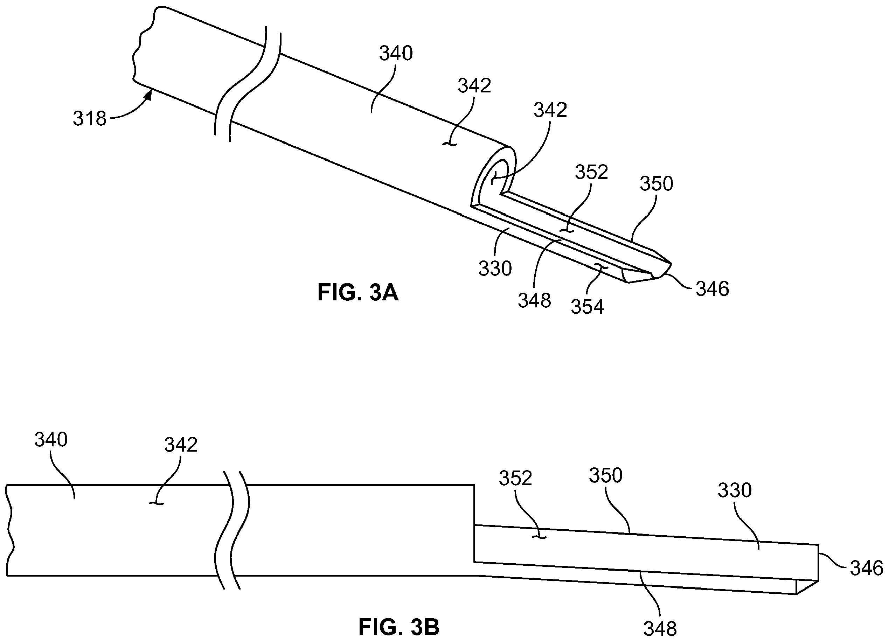

FIG. 3A is a perspective view of one embodiment of a delivery device 318 with a cylinder portion 328 and a spreader portion 330, which can be the same or similar to those delivery devices illustrated in and described with respect to FIGS. 1A-1E and 2A-2B. FIG. 3B is a side view of the delivery device 318. The cylinder portion 328 can have a relatively long and narrow cylinder body 340 with an outer surface 342 and an inner surface 344. The inner surface 344 of the cylinder body 40 can define a hollow cavity extending from a proximal end of the cylinder portion 328 to a distal end of the cylinder portion 328.

The spreader portion 330 can extend from the distal end of the cylinder portion 328. A proximal end of the spreader portion 330 can be positioned at the distal end of the cylindrical portion 328. The spreader portion 330 can have a distal edge 346 and side edges 348 and 350.

In some embodiments, the spreader portion 330 can taper to the distal edge 346. In some embodiments, the spreader portion 330 can taper to the distal edge 346 at a beveled edge. In other embodiments, the spreader portion 330 can taper to the distal edge 346 with a shape different than a bevel. In some of such embodiments, tapering the distal edge 346 can facilitate insertion of the delivery device 318.

In some embodiments, the spreader portion 330 can taper to one or both of the side edges 348 and 350. In some embodiments, the spreader portion 330 can taper to one or both of the side edges 348 and 350 at beveled edges. In other embodiments, the spreader portion 330 can taper to one or both of the side edges 348 and 350 with a shape different than a bevel. In some of such embodiments, tapering one or both of the side edges 348 and 350 can facilitate spreading of the chemotherapy drug 24 (shown in FIGS. 1A-1E). In some embodiments, the edges 346, 348, and 350 can be polished to reduce or prevent cutting of bone when the delivery device 318 is rotated.

In some embodiments, the spreader portion 330 can be curved in an arc that is less than a full 360 degrees. For example, the spreader portion 330 can be curved with the same or similar arc radius as that of the cylinder portion 328 in an arc that is less than a full cylinder. In some embodiments, the spreader portion 330 can extend in an arc that is about 120 degrees. In some embodiments, the spreader portion 330 can extend in an arc that is between about 100 degrees and about 140 degrees. In some embodiments, the spreader portion 330 can extend in an arc that is between about 80 degrees and about 190 degrees. In other embodiments, the spreader portion 330 can extend in an arc that is suitable for allowing the chemotherapy drug 24 to be pushed out of the cylinder portion 328 and be spread by the spreader portion 330 when rotated.

The spreader portion 330 can define an inner surface 352 extending between the side edges 348 and 350. The inner surface 352 can have the same or similar curvature as that of the inner surface 344 of the cylinder portion 328. The spreader portion 330 can define an outer surface 354 extending between the side edges 348 and 350 and positioned opposite and radially outward of the inner surface 352. The outer surface 354 can have the same or similar curvature as that of the outer surface 342 of the cylinder portion 328. In some embodiments, curvature of the spreader portion 330 can be different than that of the cylinder portion 328.

In some embodiments, the delivery device 318 can be formed with the cylinder portion 328 and the spreader portion 330 being a single piece. For example, the delivery device 318 can be formed from a single tube. Spreader portion 330 can be formed by cutting away a portion of the tube and machining the remaining material to form a suitable shape. For example, the spreader portion 330 can be shaped by cutting just over one-half of the tube and leaving less than one-half remaining as the spreader portion 330. Edges of the spatula portion can be beveled and polished. The spreader portion 330 can be plastically deformed to be bent at least partially outwards at an angle. In some embodiments, the delivery device 318 can be formed of a suitable medical-grade steel material. In some of such embodiments, the delivery device 318 can include steel with a sufficient amount of tin to facilitate plastic deformation. In some embodiments, the delivery device 318 can include other material suitable for the application.

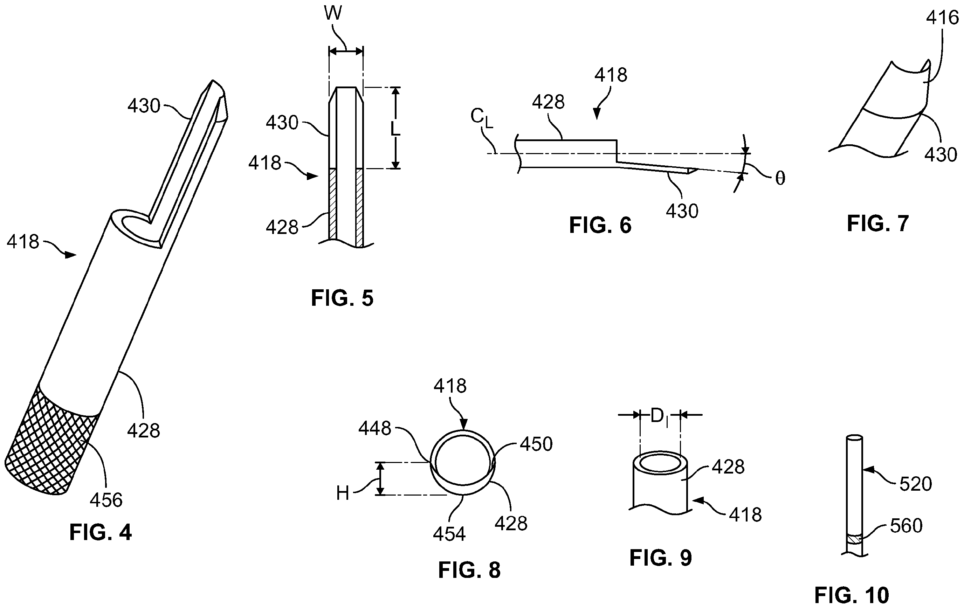

FIG. 4 is a perspective view of one embodiment of a delivery device 418 with a cylinder portion 428 and a spreader portion 430, which can be the same or similar to those delivery devices illustrated in and described with respect to FIGS. 1A-1E, 2A-2B, and 3A-3B. In the illustrated embodiment, the delivery device 418 can include a scored handle 456 to facilitate a user holding and turning the delivery device 418.

FIGS. 5-7 are side views of a distal end portion of the delivery device 418. As shown in the partial sectional side view of FIG. 5, the spreader portion 430 can have a length L in a longitudinal direction and a width W that is transverse to the longitudinal direction. In some embodiments, the spreader portion 430 can have a length L of about 16 mm. In some embodiments, the spreader portion 430 can have a length L of between about 14 mm and about 18 mm. In some embodiments, the spreader portion 430 can have a length L of between about 8 mm and about 24 mm. In some embodiments, the spreader portion 430 can have a different length L that is suitable for the application.

In some embodiments, the spreader portion 430 can have a width W of about 3.4 mm. In some embodiments, the spreader portion 430 can have a width W of about 3.04 mm. In some embodiments, the spreader portion 430 can have a width W of between about 3.0 mm and about 3.5 mm. In some embodiments, the spreader portion 430 can have a width W of between about 2.0 mm and about 5.0 mm. In some embodiments, the spreader portion 430 can have a different width W that is suitable for the application. In some embodiments, the spreader portion 430 can have a width W that is substantially equal to a width of the cylinder portion 428. In some embodiments, the spreader portion 430 can have a width W that is less than a width of the cylinder portion 428. For example, the spreader portion 430 can extend through an arc of about 120 degrees and have a width W that is less than a width of the cylinder portion 428, which can extend through 360 degrees. For example, the cylinder portion 428 can have a gauge of about 10 (about 3.404 mm outer diameter). In another example, the cylinder portion 428 can have a gauge of about 11 (about 3.048 mm outer diameter).

As shown in FIG. 6, the spreader portion 430 can extend from the cylindrical portion 328 with a configuration that is angled with respect to a centerline axis C_{L} of the cylindrical portion 328. For example, the spreader portion 430 can be angled outward at an angle Θ with respect to the centerline axis C_{L}. In some embodiments, the spreader portion 430 can be angled outward with an angle Θ of about 5 degrees. In some embodiments, the spreader portion 430 can be angled outward with an angle Θ of between about 2 degrees and about 9 degrees. In some embodiments, the spreader portion 430 can be angled outward with an angle Θ of greater than about 1 degree.

FIG. 7 illustrates a distal end 446 of the spreader portion 430 with a beveled edge. In some embodiments, the distal end 446 can be beveled at an angle that is about 35 degrees from perpendicular. In some embodiments, the distal end 446 can be beveled at an angle that is between about 30 degrees and about 40 degrees from perpendicular. In some embodiments, the distal end 446 can be beveled at an angle that is greater than about 1 degree from perpendicular.

FIG. 8 is an axial view of the distal end 446 of the delivery device 418. As shown in FIG. 8, the spreader portion 430 can be tapered at side edges 448 and 450. The spreader portion 430 can have a height H that extends from a plane defined by the side edges 448 and 450 to an outer surface 454 of the spreader portion 430. In some embodiments, the spreader portion 430 can have a height H of about 1.1 mm. In some embodiments, the spreader portion 430 can have a height H of between about 0.9 mm and about 1.3 mm. In some embodiments, the spreader portion 430 can have a height H of between about 0.3 mm and about 2.0 mm. In some embodiments, the spreader portion 430 can have a different height H suitable for the application.

FIG. 9 is a perspective view of a proximal end of the cylinder portion 428 of the delivery device 418. The cylinder portion 428 can have an inner diameter D_{I}. In some embodiments, the cylinder portion 428 can have an inner diameter D_{I} of about 2.9 mm. In some embodiments, the cylinder portion 428 can have an inner diameter D_{I} of about 2.5 mm. In some embodiments, the cylinder portion 428 can have an inner diameter D_{I} of between about 2.4 mm and about 3.0 mm. In some embodiments, the cylinder portion 428 can have an inner diameter D_{I} of between about 2.0 mm and about 3.9 mm. In some embodiments, the cylinder portion 428 can have a different inner diameter D_{I} that is about equal to or greater than that of a complementary plunger, such as the plunger 20 (shown in FIGS. 1A-1E) or a plunger 520 (shown in FIG. 10).

FIG. 10 is a perspective view of one embodiment of a plunger 520, which can be the same or similar to the plunger 20 illustrated in and described with respect to FIGS. 1A-1E. The plunger 520 can have an outer diameter that is less than or equal to the inner diameter D_{I} of the cylinder portion 428 (shown in FIG. 9). For example, in embodiments where the inner diameter D_{I} of the cylinder portion 428 is about 2.9 mm, the plunger 520 can have an outer diameter of about 2.7 mm (or approximately gauge 12: 2.769 mm). In another example, in embodiments where the inner diameter D_{I} of the cylinder portion 428 is about 2.5 mm, the plunger 520 can have an outer diameter of about 2.4 mm (r approximately gauge 13: 2.413 mm).

In some embodiments, the plunger 520 can have a length that is longer than the cylinder portion 428, and that is suitably long to allow the plunger 520 to press the chemotherapy material 24 through and out of the cylinder portion 420. For example, the plunger 520 can be as long or longer than the delivery device 418. In some of such embodiments, the plunger 520 can have one or more reference marks 560 that facilitates positioning the chemotherapy material 24 at a location suitable to be spread by the spreader portion 430 of the delivery device 418. The reference mark 560 can help prevent or reduce the tendency to push the chemotherapy material 24 all the way to the very end of a pilot hole, but instead be positioned at a location suitable for spreading. In some embodiments, the plunger 520 can have a length that is approximately equal to that of the delivery device 418 with a reference mark 560 at a location that is close to but spaced from a proximal end of the plunger 520. The reference mark 560 can be aligned with a proximal end of the delivery device 418 as an indication to the surgeon of now far the plunger 520 should be inserted to properly position the chemotherapy material 24.

In one example, the delivery device 418 and the plunger 520 can both have a length of about 17 cm. In some of such embodiments, one reference mark 560 can be located approximately 15 cm from the distal end of the plunger, or approximately 2 cm from the proximal end of the plunger. This can indicate to a surgeon using the devices that the plunger should be stopped when the distal end of the plunger 520 is approximately 2 cm from the distal end of the delivery device 418. The plunger 520 can be formed of a metal, polymer, or other material suitable for the application.

A number of embodiments of the invention have been described. Nevertheless, it will be understood that various modifications may be made without departing from the scope of the invention. For example, in some embodiments the path for accessing a vertebral body can be varied from that illustrated in FIGS. 1A-1E and 2A-2B. Additionally, while the procedure is described with respect to a vertebra, the same or similar procedure can also be applied in non-spinal bone tissue benefiting from the application. Moreover, a number of devices are shown schematically, and may be modified to have features or functions suitable for a give application. For example, one, more than one, or all features of different delivery device embodiments can be combined. Accordingly, other embodiments are within the scope of the following claims.

### Embodiments

Although the present invention is defined in the attached claims, it should be understood that the present invention can also (alternatively) be defined in accordance with the following embodiments:
1. A delivery device comprising:
   an elongate cylindrical portion; and
   a spreader portion extending from the cylindrical portion.
2. The delivery device of embodiment 1, wherein the spreader portion is configured to spread chemotherapy drug on tissue in a target site in a vertebral body.
3. The delivery device of any of the preceding embodiments, wherein the spreader portion is angled outward with respect to a centerline axis of the cylindrical portion by an angle of about 5 degrees.
4. The delivery device of any of the preceding embodiments, wherein the spreader portion is angled outward with respect to a centerline axis of the cylindrical portion by an angle of greater than about 1 degree.
5. The delivery device of any of the preceding embodiments, wherein one or more edges of the spreader portion are tapered.
6. A system comprising:
   the delivery device of any of the preceding embodiments;
   an inflatable balloon device; and
   a chemotherapy drug deliverable through the cylindrical portion and configured to be spread by the spreader portion of the delivery device.
7. The system of embodiment 6, and further comprising:
   bone cement;
   an access needle;
   a working corridor instrument;
   a plunger; and
   a drill.
8. The system of embodiment 6 or 7, wherein the system is a kyphoplasty system configured for performing a kyphoplasty procedure.
9. A system comprising:
   a means for treating cancer cells;
   a means for delivering the means for treating cancer cells to a vertebral body; and
   a means for spreading the means for treating cancer cells on a site in the vertebral body.
10. The system of embodiment 9, and further comprising:
   a means for simultaneously expanding a hole in a vertebra and pressing the means for treating cancer cells into cancellous bone along the hole.
11. The system of embodiment 10, and further comprising:
   a means for filling the hole in the vertebra.
12. A method comprising:
   delivering chemotherapy drug to a target site in a bone; and
   spreading the chemotherapy drug on bone tissue.
13. A method comprising:
   delivering chemotherapy drug through the spatula of any of embodiments 1-5 to a target site in a bone; and
   spreading the chemotherapy drug on bone tissue via the spatula of any of embodiments 1-5.

## Claims

1. A delivery device comprising:
an elongate cylindrical portion defining a hollow cavity extending from a proximal end of the cylinder portion to a distal end of the cylinder portion; and
a spreader portion extending from the distal end of the cylindrical portion.

2. The delivery device of claim 1, wherein the spreader portion is configured to spread chemotherapy drug on tissue in a target site in a vertebral body.

3. The delivery device of claim 1, wherein the spreader portion is angled outward with respect to a centerline axis of the cylindrical portion by an angle of about 5 degrees.

4. The delivery device of claim 1, wherein the spreader portion is angled outward with respect to a centerline axis of the cylindrical portion by an angle of greater than about 1 degree.

5. The delivery device of claim 1, wherein one or more edges of the spreader portion are tapered.

6. The delivery device of claim 1, wherein the spreader portion is a spatula that is angled outward with respect to a centerline axis of the cylindrical portion and is curved circumferentially with tapered edges such that the spatula is configured to suitably spread chemotherapy drug on cancellous bone.

7. The delivery device of claim 1, wherein the spreader portion has a width of between about 2.0 mm and about 5.0 mm and a length of between about 8 mm and about 24 mm.

8. The delivery device of claim 7, wherein the spreader portion has a height of between about 0.3 mm and about 2.0 mm.

9. The delivery device of claim 1, wherein the spreader portion has a width that is less than a width of the cylindrical portion and has a length that is less than a length of the cylindrical portion.

10. A system comprising:
the delivery device of any one of claims 1 to 9;
an inflatable balloon device; and
a chemotherapy drug deliverable through the cylindrical portion and configured to be spread by the spreader portion of the delivery device.

11. The system of claim 10, and further comprising:
bone cement;
an access needle;
a working corridor instrument;
a plunger; and
a drill.

12. The system of claim 10, wherein the system is a kyphoplasty system configured for performing a kyphoplasty procedure.

13. A method comprising:
delivering chemotherapy drug through the elongated cylindrical portion of the delivery device of any one of claims 1 to 9 to a target site in a bone; and
spreading the chemotherapy drug on bone tissue via the spreader portion of the delivery device of any one of claims 1 to 9 by rotating the delivery device so as to rotate the spreader portion.

14. The method of claim 13, and further comprising:
forming a working channel by inserting a needle and cannula simultaneously to a vertebra and then removing the needle;
drilling a hole through the bone tissue via a bone drill extending through the cannula;
inserting the delivery device through the cannula and into the vertebra so as to extend out both proximal and distal ends of the cannula;
removing the delivery device after the spreader portion has spread the chemotherapy drug on the bone tissue; and
inflating a balloon device to press the chemotherapy drug into the bone tissue after the spreader portion has spread the chemotherapy drug on the bone tissue.
